**Europäisches Patentamt**

**European Patent Office**

**Office européen des brevets**

(11) Veröffentlichungsnummer: **0 213 633 B1**

(12) **EUROPÄISCHE PATENTSCHRIFT**

(45) Veröffentlichungstag der Patentschrift:
18.01.89

(21) Anmeldenummer: **86112145.7**

(22) Anmeldetag: **02.09.86**

(51) Int. Cl.⁴: **C 07 C 33/025**, C 07 C 33/30, C 07 C 33/16, C 07 C 69/618, A 61 K 7/46

(54) Alkohole und Ester aus der Reaktion von Butadien mit Magnesium und Alkylhalogeniden, ihre Herstellung und deren Verwendung als Duftstoffe.

(30) Priorität: 04.09.85 DE 3531609

(43) Veröffentlichungstag der Anmeldung:
11.03.87 Patentblatt 87/11

(45) Bekanntmachung des Hinweises auf die Patenterteilung:
18.01.89 Patentblatt 89/3

(84) Benannte Vertragsstaaten:
CH DE FR GB LI NL

(56) Entgegenhaltungen:
EP-A- 0 029 603
EP-A- 0 142 812
DE-A- 2 311 040
DE-A- 2 708 048

(73) Patentinhaber: Consortium für elektrochemische Industrie GmbH, Zielstattstrasse 20, D-8000 München 70 (DE)

(72) Erfinder: Hafner, Walter, Dr. Dipl.-Chem., Birkenallee 17, D-8196 Eurasburg (DE)
Erfinder: Ritter, Peter, Brotkorbweg 60, D-8960 Kempten (DE)
Erfinder: Friedrich, Wilhelm, Ernst-Platz-Strasse 34/0, D-8000 München 50 (DE)
Erfinder: Gebauer, Helmut, Dr. Dipl-Chem., Schaffhauser Strasse 18/7, D-8000 München 71 (DE)
Erfinder: Regiert, Marlies, Schraudolphstrasse 2a, D-8000 München 40 (DE)

## Beschreibung

Die Erfindung betrifft Verbindungen, die über die Reaktion des Butadien-Magnesiums mit Isopropyl-, Benzyl- oder Cyclododecylhalogeniden zugänglich sind.

Gemäss DE-OS-2 311 040 sind bereits Reaktionsprodukte des Butadien-Magnesiums mit Isopren bekannt geworden.

Als technologischer Hintergrund sind ausserdem die Dokumente DE-A-2 708 048, worin tertiäre Alkohole und deren Verwendung als Aromastoffe für parfümierte Produkte beschrieben sind, EP-A2-142 812, worin 2-Ethyl-2-prenyl-3-hexenol und dessen Verwendung als Riechstoff beschrieben ist, und EP-A1-29 603, worin allylische Alkohole und deren Verwendung zur Synthese von Riechstoffen beschrieben sind, zu nennen.

Es wurde nun gefunden, dass eine Auswahl von Verbindungen, die über die Reaktion des Butadien-Magnesiums mit Isopropyl-, Benzyl- oder Cyclododecyl-Halogeniden und weiterer Derivatisierung zugänglich sind, ausgezeichnete Duftstoff- und Fixateureigenschaften aufweisen.

Gegenstand der Erfindung sind:

5-Methyl-3-vinyl-hexan-2-ol
2-Benzyl-3-buten-1-ol

3-Benzyl-4-pentensäuremethylester
3-Benzyl-4-pentensäureethylester

1-Cyclododecyl-3-buten-1-ol
1-Cyclododecyl-3-buten-2-ol
2-Cyclododecyl-3-buten-1-ol
4-Cyclododecyl-2-buten-1-ol

Im Hinblick auf die erfindungsgemässen Cyclododecyl-Verbindungen sind Gemische bevorzugt, insbesondere Gemische aus cis- und trans-4-Cyclododecyl-2-buten-1-ol, 1-Cyclododecyl-3-buten-2-ol und 2-Cyclododecyl-3-buten-1-ol, sowie Gemische aus 1-Cyclododecyl-3-buten-2-ol, 2-Cyclododecyl-3 buten-1-ol und 1-Cyclododecyl-3-buten-1-ol.

Die erfindungsgemässen Verbindungen sind zugänglich durch Umsetzen von Butadien-Magnesium mit Isopropylhalogenid, Benzylhalogenid oder Cyclodecylhalogenid und anschliessender Weiterverarbeitung der erhaltenen Alkylbutenylmagnesium-Halogenide. Unter Weiterverarbeitung wird erfindungsgemäss verstanden:

a) Die Oxydation der Alkylbutenylmagnesium-Halogenide mit Sauerstoff und anschliessende Hydrolyse zu den entsprechenden Alkoholen oder
b) Die Umsetzung der Alkylbutenylmagnesium-Halogenid-Verbindungen mit $CO_2$, Hydrolyse und anschliessende Veresterung der erhaltenen Säuren oder
c) Umsetzung des Alkylbutenylmagnesium-Halogenids mit Acetaldehyd und anschliessende Hydrolyse zum entsprechenden Alkohol.

Das als Ausgangsmaterial benötigte Butadienmagnesium wird durch Reaktion von Butadien mit metallischem Magnesium, das ggf. mittels Halogeniden wie Alkylbromid, Allylchlorid und anderen aktiviert wurde, gewonnen. Tetrahydrofuran dient als Lösungsmittel. Die Reaktionstemperaturen betragen vorzugsweise 20°C bis 65°C.

Die Umsetzungen des Butadien-Magnesiums mit den im Rahmen der Erfindung eingesetzten Alkylhalogeniden erfolgt zweckmässigerweise im gleichen Lösungsmittel. Es werden zumeist in etwa äquimulare Mengen an Butadien-Magnesium und Alkylhalogenid eingesetzt. Technisch sinnvoll sind Ansätze von 0,8 mol bis 1 mol Alkylhalogenid pro Mol Butadien-Magnesium. Die Reaktionstemperaturen liegen im Bereich von 20 bis 65°C, insbesondere 40 bis 60°C.

Als Alkylhalogenide kommen im Rahmen der Erfindung die Chloride Bromide und Jodide, insbesondere die Chloride oder Bromide der Alkylreste Isopropyl, Cyclododecyl und Benzyl in Betracht.

Diese Umsetzungen werden erfindungsgemäss oftmals in Gegenwart von katalytischen Mengen an Übergangsmetall-Verbindung durchgeführt. Beispiele hierfür sind $PdCl_2$, $P(C_6H_5)_3$, $MnCl_2$, $ZnCl_2$, CuJ und andere. Die genannten Übergangsmetallverbindungen dirigieren sekundäre Alkylreste überwiegend in die 1-Stellung des Butadiens. Falls die Reaktion der Alkylreste in 2-Stellung erwünscht ist, wird vorteilhafterweise in Gegenwart katalytischer Mengen von Titansäureestern, wie beispielsweise Tetrabutyltitanat gearbeitet.

Die weitere Umsetzung der Alkylbutenylmagnesium-Halogenide kann analog den an sich bekannten Reaktionen von Grignard-Verbindungen erfolgen:

a) Es wird mit Sauerstoff oder sauerstoffhaltigen Gasen insbesondere mit $CO_2$-freier Luft oxydiert bei Reaktionstemperaturen vorzugsweise −25°C bis·+10°C und anschliessend hydrolysiert.
b) Es wird mit vorzugsweise gasförmigem $CO_2$ umgesetzt und die nach saurer Hydrolyse erhaltene Säure in an sich bekannter Weise verestert.
c) Es wird mit Acetaldehyd umgesetzt und anschliessend sauer hydrolysiert.

Das Verfahren zur Herstellung von 5-Methyl-3-vinyl-hexan-2-ol ist dadurch gekennzeichnet, dass

a) Butadien-Magnesium mit Isopropylchlorid, -bromid oder -jodid umgesetzt wird,
b) Acetaldehyd an das gemäss a) erhaltene Isoheptenyl-Magnesium-Halogenid addiert wird,
c) Das Reaktionsprodukt gemäss b) in Gegenwart von Säure hydrolysiert wird.

Das Verfahren zur Herstellung von 2-Benzyl-3-buten-1-ol ist dadurch gekennzeichnet, dass

a) Butadien-Magnesium mit Benzylchlorid oder -bromid umgesetzt wird,
b) Das gemäss a) erhaltene Benzyl-buten-magnesium-halogenid mit Sauerstoff oxydiert wird und
c) Das Reaktionsprodukt gemäss b) einer sauren Hydrolyse unterworfen wird.

Das Verfahren zur Herstellung von 3-Benzyl-4-pentensäure-alkylester, wobei Alkyl Methyl oder Äthyl, ist dadurch gekennzeichnet, dass

a) Butadien-Magnesium mit Benzylchlorid- oder -bromid umgesetzt wird,
b) Das gemäss a) erhaltene Benzylbuten-magnesium-halogenid mit $CO_2$ behandelt wird, und

c) Das Reaktionsprodukt b) hydrolysiert und in an sich bekannter Weise in den Methyl- oder Äthylester übergeführt wird.

Das Verfahren zur Herstellung von 1-Cyclododecyl-3-buten-1-ol, 1-Cyclododecyl-3-buten-2-ol, 2-Cyclododecyl-3-buten-1-ol und 4-Cyclododecyl-2-buten-1-ol ist dadurch gekennzeichnet, dass

a) Butadien-Magnesium, ggf. in Gegenwart von Übergangsmetallhalogenid mit Cyclododecylchlorid, -bromid oder -jodid umgesetzt wird,

b) Das gemäss a) erhaltene Cyclododecyl-buten-magnesium-halogenid mit Sauerstoff oxidiert wird, und

c) Das gemäss b) erhaltene Produkt einer saueren Hydrolyse unterworfen wird.

Die erfindungsgemässen Verbindungen finden Verwendung als Duftstoffe. Sie werden zur Parfümierung kosmetischer und technischer Produkte eingesetzt. Die erfindungsgemässen Duftstoffe belegen ein breites Spektrum verschiedenster Duftnoten von süss-blumigen Noten bis zu holzigen Noten mit Ambra-Unterton. Die Cyclododecylbutenole werden erfindungsgemäss bevorzugt im Gemisch eingesetzt. Die einzelnen Komponenten können dabei in weiten Bereichen variieren: Die hauptsächlichen Duftträger sind 1-Cyclododecyl-3-buten-1-ol, 2-Cyclododecyl-3-buten-1-ol und 1-Cyclododecyl-3-buten-2-ol. Sie weisen balsamische, an Zedernöl erinnernde Holznoten auf. Der Anteil der drei genannten Cyclododecylbutenole kann — bezogen auf das Gesamtgemisch der Cyclododecylbutenole — variieren in der praktischen Anwendung etwa im Bereich von 5 - 80 Gew.-%. Cis- und trans-4-Cyclododecyl-2-buten-1-ol weisen einen nicht intensiven, fettiggrünen Holzgeruch auf. Sie wirken im wesentlichen fixierend und modifizierend.

Die Erfindung wird nun anhand von Beispielen näher erläutert:

*Beispiel 1*

*Herstellung von 5-Methyl-3-vinyl-hexan-2-ol*

In einem 2 l 4-Halskolben wurden 50 g Magnesium-Pulver und 100 ml trockenes Tetrahydrofuran vorgelegt. Unter Spülen des Reaktionsgefässes mit Butadien und Rühren wurde bei 40°C durch Zugabe von 6 ml Allylchlorid die Reaktion in Gang gebracht und der Ansatz allmählich mit 200 ml Tetrahydrofuran verdünnt. Parallel dazu wurde eine erwärmte Mischung aus 2 g Palladiumchlorid und 5,9 g Triphenylphosphin in 120 ml Tetrahydrofuran langsam zu der dunkelgrünen Reaktionsmischung gegeben, die nun eine schwarzbraune Farbe annahm. Unter weiterer Butadienzufuhr wurde nun eine Mischung aus 157 g Isopropylchlorid mit 300 ml Tetrahydrofuran im Verlauf von 6½ Stunden zugetropft. Die Temperatur wurde dabei im Bereich von 55 bis 60°C gehalten.

Danach wurde die Apparatur mit Stickstoff gespült und abgekühlt. Anschliessend wurde ein Gemisch von 90 g frisch destilliertem Acetaldehyd und 150 ml Tetrahydrofuran der Reaktionsmischung zugetropft. Die Reaktionstemperatur wurde zwischen 10 und 20°C gehalten. Schliesslich wurde das Reaktionsgemisch in Eis eingerührt und die organische Phase abgetrennt. Die wässrige Phase wurde mit 10 n Salzsäure angesäuert und mit Diethyläther extrahiert. Die vereinigten organischen Phasen wurden noch mit Wasser extrahiert, schliesslich über Aktivkohle filtriert und mit festem Kaliumcarbonat getrocknet. Die fraktionierte Destillation über eine 30 cm lange Füllkörperkolonne mit Drahtwendeln ergab im Temperaturintervall von 67 - 72°C bei 16 mbar 120 g Destillat. Es wurde ein Stereoisomerengemisch von 5-Methyl-3-vinyl-hexan-2-ol erhalten.

Geruch: Herb-grüner Duftkomplex mit minzigwürziger Beinote, das mit dem Geruch von Chrysanthemen- u. Buccoblätter vergleichbar ist.

*Beispiel 2*

*Herstellung von 2-Benzyl-3-buten-1-ol*

In einem 2 l 4-Halskolben wurden 50 g Magnesium-Pulver unter Butadien-Atmosphäre mit 6 ml Allylchlorid in 80 ml Tetrahydrofuran bei 40°C zur Reaktion gebracht. Unter kräftigem Rühren wurden nun bei ständiger Sättigung mit Butadien innerhalb von 30 Minuten 400 ml Tetrahydrofuran zugeführt. Danach wurden unter leichter Rührung soviel an 50 gew.%iger Benzylchlorid/Tetrahydrofuranmischung zugetropft, dass die Reaktionslösung noch eine leicht gelb-grüne Färbung aufwies. Anschliessend wurde wiederum eine ½ Stunde kräftig gerührt, um danach die entstandene Butadien-Magnesium-Verbindung wieder mit weiterem Benzylchlorid/Tetrahydrofurangemisch umzusetzen. Diese Prozedur wurde solang wiederholt, bis das Magnesium-Pulver aufgelöst war. Die Reaktionstemperatur wurde zwischen 38 und 40°C gehalten.

Es wurde nun mit Stickstoff gespült, auf −20°C abgekühlt und $CO_2$-freie trockene Luft eingeblasen, bis keine Wärmetönung mehr zu erkennen war. Das Reaktionsgemisch wurde schliesslich in Eis eingerührt und die organische Phase abgetrennt. Die wässrige Phase wurde mit 10 n Salzsäure angesäuert und mit Diethyläther extrahiert. Die vereinigten organischen Phasen wurden noch mit Wasser extrahiert und die organische Phase mit festem Kaliumcarbonat getrocknet. Nach Abziehen des Lösungsmittels ergab die fraktionierte Destillation über eine 30 cm lange Füllkörperkolonne mit Stahlwendeln im Temperaturintervall von 70 bis 74°C bei 0,12 mbar 160 g 2-Benzyl-3-buten-1-ol.

Geruch: Strenge grüne Blumennote von Hyacinthen-Charakter, zudem rosig, fruchtig.

*Beispiel 3*

*Herstellung von Cyclododecylbutenolen*

In einem 2 l 4-Halskolben wurden 50 g Magnesium-Pulver mit trockenem Tetrahydrofuran überschichtet und unter Einleiten von Butadien mit 2 ml Äthylbromid aktiviert. Nach dem Anspringen der Reaktion wurde portionsweise mit 200 ml Tetrahydrofuran verdünnt und unter kräftigem Rühren weiterhin Butadien eingeleitet. Das Reaktionsgemisch färbte sich dunkelgrün. Die Reaktionstemperatur betrug 60°C. Unter weiterer Butadienzufuhr wurde nun im Verlauf von 6 Stunden eine Mischung aus 400 g Cyclododecylchlorid und 900 ml Tetrahydrofuran zugetropft. Danach wurde noch eine weitere Stunde bei 60°C gerührt. Anschliessend wurde die Reaktionsmischung 12 Stunden bei Raumtemperatur unter Luftausschluss stehengelassen.

Das Reaktionsgemisch wurde anschliessend abgekühlt und im Temperaturintervall von −18 bis −22°C solange $CO_2$-freie trockene Luft eingeleitet (ca. 3,5 Stunden), bis keine exotherme Reaktion mehr zu erkennen war. Zur Hydrolyse wurde die Reaktionsmischung in Eis eingerührt. Die organische Phase wurde abgetrennt, die wässrige Phase mit 10 n Salzsäure angesäuert und mit Äther extrahiert. Die vereinigten organischen Phasen wurden noch mit Wasser extrahiert und die organische Phase schliesslich über Kaliumcarbonat getrocknet. Nach Abtrennung der Lösungsmittel wurde durch fraktionierte Destillation an einer 30 cm langen Füllkörperkolonne im Temperaturintervall von 105 - 135°C bei 0,12 mbar 108 g eines Gemisches von Cyclododecylbutenolen erhalten. Die chromatographische Auftrennung ergab folgende Isomerenverteilung:

63 Gew.-% cis- und trans-4-Cyclododecyl-2-buten 1 ol
24 Gew.-% 2-Cyclododecyl-3-buten-1-ol
10 Gew.-% 1-Cyclododecyl-3-buten-2-ol
0,5 Gew.-% 1-Cyclododecyl-3-buten-1-ol
Rest im wesentlichen Cyclododekanol

Geruch des isomeren Gemisches: Stark ambrierte Holznote mit blumiger Komponente.

### Beispiel 3a

*Herstellung von Cyclododecylbutenolen*

In einem 1 l 4-Halskolben wurde in einer Butadien-Atmosphäre mit 21 g Magnesiumpulver, 40 ml THF und 3 ml Allylchlorid auf etwa 50°C erwärmt, bis die Grignard-Reaktion in Gang kam. Unter ständiger Butadien-Zufuhr bei Normaldruck und kräftigem Rühren wurden 30 Minuten 200 ml THF zugetropft. Dann wurde die Rührgeschwindigkeit soweit zurückgenommen, dass sich das Magnesiumpulver absetzen konnte. Nun wurde eine Mischung von Cyclodecylbromid mit THF zugetropft, bis die gelbgrüne Farbe der Mischung zu verblassen begann. Nach ca. 5 Minuten Wartezeit wurde wieder 30 Minuten schnell gerührt. Bei geringer Rührgeschwindigkeit wurde dann wieder Cyclododecylbromid zugegeben. Mit Unterbrechung über Nacht wurde dieses Wechselspiel 12 Stunden fortgeführt, bis etwa ein Mol Cyclododecylbromid verbraucht war. Die Reaktionstemperatur wurde durch ein Wasserbad bei 40 bis 45°C gehalten.

Anschliessend wurde der Ansatz unter $N_2$-Spülung auf −20°C abkühlt und im Verlauf von 4 Stunden mit in die Reaktionsmischung eingeblasener Luft oxidiert. Nach üblicher Zersetzung, Aufarbeitung und Entfernung der Lösungsmittel enthielt die Reaktionsmischung nach Gaschromatogramm:

1,7% Cyclododecanol
0,5% 1-Cyclododecyl-3-buten-1-ol
15,5% 1-Cyclododecyl-3-buten-2-ol
2,7% 2-Cyclododecyl-3-buten-1-ol
31 % 4-Cyclododecyl-2-buten-1-ol (cis und trans)

Der Rest betand unter anderem aus nicht umgesetztem Cyclododecylbromid, Cyclododecylbutene und -octadiene, Cyclododecyloctadienole, sowie Biscyclododecyl und von Butadienoligomeren abgeleiteten Alkoholen ($C_8$, $C_{12}$, $C_{16}$ und höher).

Destillation über eine 30 cm lange Füllkörperkolonne unter einem Druck von 0,05 Torr ergab von 103 bis 122°C 56 g Destillat mit folgender Zusammensetzung:

38 % 1-Cyclododecyl-3-buten-2-ol
48 % 4-Cyclododecyl-2-buten-1-ol
8 % 2-Cyclododecyl-3-buten-1-ol
1,2% 1-Cyclododecyl-2-buten-1-ol

Geruch: Ähnlich wie im Beispiel 3, wobei aber die blumige Komponente zurücktritt.

Führt man den gleichen Versuch in Gegenwart von 7 g $ZnBr_2$ durch, so wird die Bildung von 2-Cyclododecyl-3-buten-1-ol und 1-Cyclododecyl-3-buten-1-ol weitgehend unterdrückt.

### Beispiel 4

*Herstellung von 3-Benzyl-4-pentensäureethylester*

In einem 2 l 4-Halskolben wurden 55 g Magnesium-Pulver mit 70 ml Tetrahydrofuran bedeckt und nach Spülen der Apparatur mit Butadien mit 3 ml Allylchlorid aktiviert. Unter fortwährender Butadienzufuhr wurden im Verlauf von 15 Minuten 250 ml Tetrahydrofuran zugetropft, wobei eine Temperatur von 50 bis 55°C eingehalten wurde. Dieser Vorlage wurde nun innerhalb von 7 Stunden eine Mischung aus 250 g Benzylchlorid und 250 ml Tetrahydrofuran zudosiert. Anschliessend wurde mit Stickstoff gespült und auf Raumtemperatur abgekühlt. Die Lösung wurde nun unter Stickstoff langsam in einen 2. Reaktionskolben übergeführt, in dem sich 200 ml, mit $CO_2$-gesättigtes Tetrahydrofuran fanden. Durch gleichzeitiges Einleiten von $CO_2$ wurde immer ein Überschuss davon angeboten. Die Reaktionstemperatur betrug 10°C. Zur Hydrolyse wurde das Reaktionsgemisch schliesslich auf Eis gegossen und mit 10 n Salzsäure angesäuert. Anschliessend wurde mit Äther extrahiert. Der ätherische Extrakt wurde nun wiederum unter Kühlung mit 2 n NaOH extrahiert. Nach dem Ansäuern der wässrigen Phase wurde erneut mit Äther extrahiert. Die ätherische Phase wurde schliesslich mit Wasser extrahiert und mit Calciumchlorid getrocknet. Nach Abziehen des Lösungsmittels verblieben 387 g Rohprodukt (im wesentlichen 3-Benzyl-4-Pentensäure).

Zur Veresterung wurden 330 g des Rohproduktes mit 2 g para-Toluolsulfonsäure versetzt und mit Cyclohexan Ethanol unter azeotroper Wasserabscheidung zur Reaktion gebracht. Nach Entfernen der para-Toluolsulfonsäure mit Natriumbicarbonatlösung wurde die organische Phase getrocknet und über eine 30 cm lange Füllkörperkolonne mit Stahlwendeln destilliert. Die fraktionierte Destillation erbrachte im Temperaturintervall von 80 - 83°C bei 0,12 mbar 293 g 3-Benzyl-4-pentensäureethylester.

Geruch: Fruchtig blumiger Duftkomplex von Honig, schwarzen Johannisbeeren Gardenie, Tabak.

*Beispiel 5*

*Herstellung von 3-Benzyl-4-pentensäuremethylester*

30 g 3-Benzyl-4-pentensäure (gemäss Beispiel 4) wurden mit 100 ml Methanol und 0,5 g para-Toluolsulfonsäure eine Stunde unter Rückfluss gekocht. Danach wurden noch weitere 5 Stunden wasserhaltiges Methanol abdestilliert und durch trockenes Methanol ersetzt. Schliesslich wurde mit Äther versetzt, mit wässriger Natriumbicarbonat-Lösung säurefreigewaschen und die organische Phase getrocknet und destilliert.

Die fraktionierte Destillation erbrachte bei 60°C/0,18 mbar 25 g 3-Benzyl-4-pentensäuremethylester.

Geruch: Fruchtig, warm-krautig, blumig mit starker Honignote und Aspekten von Ananas, Rose.

*Beispiel 6*

|  | *Gewichtsteile* | |
|---|---|---|
| Parfumöl mit Hyacinthen-Note | a | b |
| Hexenylacetat-cis-3, 10% in DPG* | 20 | 20 |
| Indol, 1% in DPG* | 40 | 40 |
| Galbanumöl, 10% in DPG* | 60 | 60 |
| Phenylacetaldehyd, 10% in DPG* | 85 | 85 |
| Methyl-dihydrojasmonat | 70 | 70 |
| 1,1-Dimethoxy-2,2,5-trimethyl-4-hexan (Amarocit)[1] | 20 | 20 |
| Citronenöl | 45 | 45 |
| Orangenöl bras. | 45 | 45 |
| Petitgrainöl Paraguay | 20 | 20 |
| Linalylacetat | 75 | 75 |
| Linalool | 55 | 55 |
| Hydroxycitronellal | 45 | 45 |
| Phenylethanol | 130 | 130 |
| Dimethylbenzylcarbinol | 10 | 10 |
| 4-Acetoxy-3-pentyltetrahydropyran + 1,3-Diacetoxynonan (=Zaswalia) | 45 | 45 |
| Eugenol | 10 | 10 |
| cis-3-Hexenylsalicylat | 35 | 35 |
| Zimtalkohol | 40 | 40 |
| 5-Cyclohexadecenon | 40 | 40 |
| Dipropylenglycol | 80 | – |
| 2-Benzyl-3-buten-1-ol | – | 80 |
|  | 1000 | 1000 |

DPG: Dipropylenglycol
[1]) Herkunft: Consortium für elektrochemische Industrie.

Gibt man zum Parfumöl a mit seinem Hyacinthen-ähnlichen Duftkomplex 8% des erfindungsgemässen Alkohols, so wird der grünblumige Duft charakteristischer, seine Intensität und Süsse nehmen zu, er erinnert an natürliche Hyacinthenblüten.

**Patentansprüche**

1. 5-Methyl-3-vinyl-hexan-2-ol, 2-Benzyl-3-buten-1-ol, 3-Benzyl-4-pentensäuremethylester, 3-Benzyl-4-pentensäureäthylester, 1-Cyclododecyl-3-buten-1-ol, 1-Cyclododecyl-3-buten-2-ol, 2-Cyclododecyl-3-buten-1-ol, 4-Cyclododecyl-2-buten-1-ol.

2. Verwendung der Verbindungen nach Anspruch 1 als Duftstoffe.

**Claims**

1. 5-Methyl-3-vinyl-hexan-2-ol, 2-benzyl-but-3-en-1-ol, methyl 3-benzyl-4-pentenoate, ethyl 3-benzyl-4-pentenoate, 1-cyclododecyl-but-3-en-1-ol, 1-cyclododecyl-but-3-en-2-ol, 2-cyclododecyl-but-3-en-1-ol and 4-cyclododecyl-but-2-en-1-ol.

2. Use of the compounds according to claim 1 as fragrances.

**Revendications**

1. 5-Méthyl-3-vinyl-hexanol-2, 2-benzyl-3-buténol-1, 3-benzyl-4-penténoate de méthyle, 3-benzyl-4-penténoate d'éthyle, 1-cyclododécyl-3-buténol-1, 1-cyclododécyl-3-buténol-2, 2-cyclododécyl-3-buténol-1 et 4-cyclododécyl-2-buténol-1.

2. L'utilisation comme parfums des composés selon la revendication 1.